# EUROPEAN PATENT APPLICATION

(11) **EP 4 744 670 A1**
(43) Date of publication of application: **20.05.2026**
(21) Application number: 24838815.9
(22) Date of filing: 10.07.2024
(51) Int. Cl.: A61K 31/765, A61K 31/74, A61L 27/18, A61L 27/50, A61P 25/00

(54) **POLYLACTIC ACID AND USE OF COPOLYMER THEREOF IN PREPARATION OF DRUG FOR PROMOTING NERVE GROWTH AND REPAIR**

(30) Priority: 12.07.2023 CN 202310854812
(71) Applicant: Changchun Sinobiomaterials Co., Ltd., Changchun, Jilin 130000 (CN)
(72) Inventor: WANG, Jinyue, Changchun, Jilin 130000 (CN); ZHUANG, Xiuli, Changchun, Jilin 130000 (CN); ZHANG, Tianhui, Changchun, Jilin 130000 (CN)
(74) Representative: Colombo, Stefano Paolo
(86) International application number: PCT/CN2024/104589
(87) International publication number: WO 2025/011563

(57) **Abstract**

Provided are a polylactic acid and a use of a copolymer thereof in the preparation of a drug for promoting nerve growth and repair. The molecular weight of the polylactic acid and the copolymer thereof is 400-300 k Da. The drug has the following effects: 1) promoting the growth of nerve cells and nerve tissue; 2) promoting repair of nerve cells and nerve tissue; and 3) treating or preventing a peripheral nerve injury, a spinal cord injury, peripheral neuropathy and neuritis. The molecular weight is preferably 5000-100 k Da. It is found for the first time that the polylactic acid and the copolymer thereof have a protective effect on nerves, and can promote nerve growth and a nerve injury repair.

## Description

### CROSS-REFERENCE TO RELATED APPLICATIONS

The present application claims priority to Chinese patent application No. 202310854812.1, filed on July 12, 2023, entitled "Polylactic Acid and Use of Copolymer Thereof in Preparation of Drug for Promoting Nerve Growth and Repair", which is incorporated herein by reference in its entirety.

### TECHNICAL FIELD

The present invention relates to the field of polymer medicine technology, and particularly relates to the use of polymers comprising lactic acid repeating units in the preparation of drugs for promoting nerve growth and repair.

### BACKGROUND

Polylactic acid (PLA) has the molecular formula (C₃H₄O₂)ₙ and is mainly prepared by lactic acid polymerization or lactide ring-opening polymerization. Its structural formula is

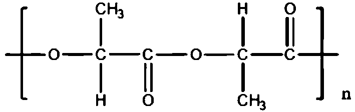

PLA and the copolymer thereof not only possess excellent mechanical strength and chemical stability, but also exhibit good biocompatibility and biodegradability. In recent years, a great deal of research has been conducted both domestically and internationally on its application in biomedicine. It has been widely used in surgical sutures, bone repair materials, controlled-release drug systems, and tissue functional scaffolds (such as artificial bone and artificial skin).

Among others, biodegradable polylactic acid microparticles with good biocompatibility (trade name SculptraTM) were approved by the U.S. FDA in 2004 for filling in sagging skin areas caused by facial fat atrophy in AIDS patients. At the same time, clinicians also use it for age-related lipoatrophy and localized lipoatrophy in healthy individuals. In 2009, the U.S. FDA officially approved poly-L-lactic acid fillers for use in improving nasolabial folds. The mechanism of action of poly-L-lactic acid fillers is achieved through stimulating collagen production. The initial filling effect can last for about 1 week. After that, the poly-L-lactic acid filler is phagocytosed and dissolved by macrophages in the body, destroying its polymeric state. Finally, it is degraded into lactic acid, water and carbon dioxide through non-enzymatic hydrolysis. The residual lactic acid at the injection site stimulates the surrounding tissue to produce collagen. Several months later, the dermis at the injection site gradually thickens, eventually achieving the cosmetic effect.

The non-patent literature "Materials for peripheral nerve repair constructs: Natural proteins or synthetic polymers?" discloses a method for repairing nerve injury, including using electrospinning to make scaffolds or conduits from materials such as polylactic acid and using nerve regeneration agents (such as neurotrophic factors) to promote the repair of nerve function, where the polylactic acid scaffolds or conduits simply play a supporting, guiding, and bridging role for the nerve.

However, there are no reports in the prior art regarding whether polylactic acid and the copolymer thereof can be directly used as polymeric drugs for neuroprotection, repair and regeneration.

### SUMMARY OF THE INVENTION

To address the shortcomings of the prior art, the present invention provides the use of a polylactic acid and a copolymer thereof in the preparation of a drug for promoting nerve growth and repair, offering a novel approach for neuroprotection, repair, and regeneration.

An objective of the present application is to provide use of a polylactic acid and a copolymer thereof in the preparation of a drug for promoting nerve growth and repair, wherein the molecular weight of the polylactic acid and the copolymer thereof is 400-300 k Da, and the drug has any one of the following effects 1) to 3):
1) promoting the growth of nerve cells and nerve tissue;
2) promoting repair of nerve cells and nerve tissue;
3) treating or preventing a peripheral nerve injury, a spinal cord injury, peripheral neuropathy, and neuritis;
preferably, the molecular weight is 5000-100 k Da.

Furthermore, the polylactic acid and the copolymer thereof are from the following combination: poly-L-lactic acid, poly-D,L-lactic acid, and copolymers comprising L-lactic acid but not D-lactic acid.

Furthermore, the crystallinity of the poly-L-lactic acid is 0~85%, preferably 10~40%, and its molecular weight distribution is 1~3, preferably 1.1~1.8.

Furthermore, the concentration of L-lactic acid in the poly-D,L-lactic acid is at least 50%; more preferably, the ratio of L-lactic acid to D-lactic acid in the poly-D,L-lactic acid is about 1:1.

Furthermore, the concentration of L-lactic acid in the copolymer comprising L-lactic acid but not D-lactic acid is at least 20%.

Preferably, the copolymer comprising L-lactic acid but not D-lactic acid includes one or more of poly(lactic acid-glycolic acid) copolymer, poly(lactic acid-ethylene glycol) copolymer, poly(ethylene glycol-lactic acid-glycolic acid) copolymer, polylactic acid-chitosan copolymer, and poly(lactide-caprolactone) copolymer.

Furthermore, the polylactic acid and the copolymer thereof exist in the form of a formulation.

Furthermore, the formulation includes microspheres, micelles, and gels.

Preferably, the microspheres are spherical and have a smooth surface; the particle size distribution of the microspheres is 1-100 µm, preferably 30-60 µm.

Furthermore, the formulation further comprises excipients, and the excipients include surfactants and stabilizers.

The surfactant includes one or more of polyethylene glycol, sodium dodecyl sulfonate, Tween, and Span; and the stabilizer includes one or two of carboxymethyl cellulose and mannitol.

The formulation further comprises a first active ingredient having a neuroprotective effect, and preferably, the first active ingredient includes one or more of piracetam, aniracetam, oxiracetam, methylcobalamin, adenosylcobalamin, gangliosides, mannitol, edaravone, methylcobalamin, rapamycin, nerve growth factor, and vitamin B.

Furthermore, the concentration of L-lactic acid in the polylactic acid and the copolymer thereof in the formulation during at least one dosing period is 5~2500 mmol/L, preferably 100~1200 mmol/L.

In the present invention, the term "smooth surface" means that the surface of 10 spheres has no more than 50 protrusions with a height of 1 to 3 µm , and no protrusions greater than 5 µm .

Compared with the prior art, the present invention has the following beneficial effects:
(1) The present invention is the first to discover that polymers comprising lactic acid repeating units have protective and repairing effects on nerves, and can promote nerve growth and the repair of nerve injuries.
(2) The microspheres of the polylactic acid and the copolymer thereof prepared in the present invention can reduce the level of malondialdehyde in the spinal cord at the severed ends after complete transection injury of the spinal cord, reduce the damage caused by lipid peroxidation, and thus protect the spinal cord tissue. The drug of the present invention provides early protection of the complete transection of the spinal cord, thereby extending the time window for early treatment.
(3) The microspheres of the polylactic acid and the copolymer thereof prepared in the present invention can achieve the effect of sustained release of lactic acid, improve the therapeutic effect and prolong the action time.
(4) The microspheres of the polylactic acid and the copolymer thereof prepared in the present invention reduce cytotoxicity and improve cell survival rate.

### BRIEF DESCRIPTION OF THE DRAWINGS

Fig. 1 is an SEM image of the PLLA microspheres in Example 1 of the present invention.
Fig. 2 is an SEM image of the PLGA microspheres in Example 2 of the present invention.
Fig. 3 shows the lactic acid release results of the PLLA microspheres and PLGA microspheres in Example 3 of the present invention.
Fig. 4 shows the results of cell survival rate of the PLLA microspheres and lactic acid in Example 4 of the present invention.
Fig. 5 shows the repair effect after nerve injury in Example 6 of the present invention.

### DETAILED DESCRIPTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise indicated, the raw materials and reagents used in the following examples are commercially available or may be prepared by known methods. Poly(lactic acid-glycolic acid) (ratio of LA to GA 75:25, molecular weight 15000). Poly-L-lactic acid (PLLA), a crystalline polymer.

### Example 1: Preparation of polylactic acid microspheres

9 g of poly-L-lactic acid (PLLA) (molecular weight 15000 g/mol, molecular weight distribution 1.1, crystallinity 30%) was dissolved in 135 mL of dichloromethane. The polymer solution was then added to 1800 mL of an aqueous solution of polyvinyl alcohol with a mass concentration of 0.5%. After emulsification at 3000 rpm/min for 10 min, the mixture was further stirred at 500 rpm for 3 h to remove dichloromethane, finally giving poly-L-lactic acid microspheres.

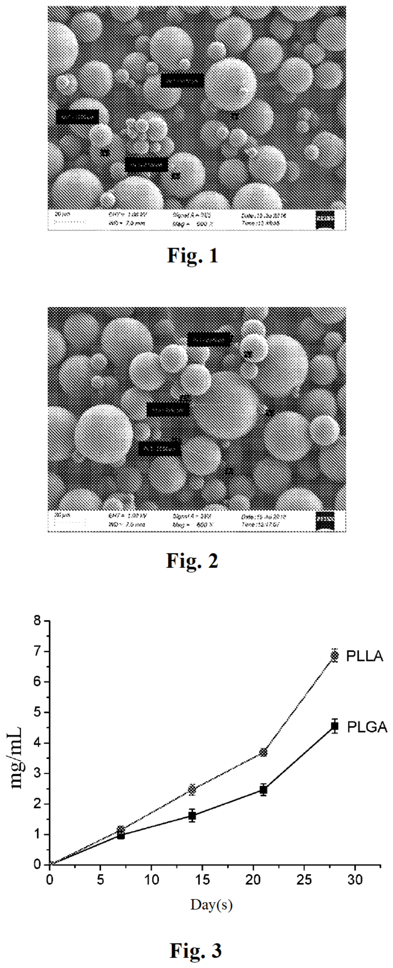

The resulting PLLA microspheres were observed using a scanning electron microscope and their particle size was calculated. The picture of the microspheres is shown in Fig. 1. The particle size range of the microspheres is 3.72-33.12µm.

### Example 2: Preparation of poly(lactic acid-glycolic acid) microspheres

9 g of poly(lactic acid-glycolic acid) (PLGA) (molecular weight 15000 g/mol, molecular weight distribution 1.1, crystallinity 30%) was dissolved in 135 mL of dichloromethane. The polymer solution was then added to 1800 mL of an aqueous solution of polyvinyl alcohol with a mass concentration of 0.5%. After emulsification at 3000 rpm/min for 10 min, the mixture was further stirred at 500 rpm for 3 h to remove dichloromethane, finally giving poly(lactic acid-glycolic acid) microspheres.

The resulting polylactic acid microspheres were observed using a scanning electron microscope and their particle size was calculated. The picture of the PLGA microspheres is shown in Fig. 2. The particle size range of the microspheres is 2.94-36.92µm.

### Example 3: Release of L-lactic acid from polylactic acid microspheres and polylactic acid copolymer microspheres

12 portions (70 mg each) of PLLA and PLGA microspheres prepared in Examples 1 and 2 were weighed, respectively. Each portion was suspended in 10 ml of phosphate buffer of pH 7.4, and then placed on a 37°C constant temperature water bath shaker and shaken at 60 rpm/min. 3 samples were taken every 7 days and the liquid portion was collected.

The change in lactic acid concentration in the medium over time was determined by high performance liquid chromatography. Chromatographic conditions: mobile phase, 0.1% aqueous solution of H₃PO₄; detection wavelength, 210 nm; flow rate, 1.0 mL/min; and injection volume, 10 µL. The experimental results are shown in Fig. 3. As can be seen from the figure, the concentration of lactic acid produced by the degradation of microspheres showed a steady increasing trend in the first 21 days of degradation. After that, lactic acid was released rapidly, indicating that the degradation of microspheres accelerated after 21 days of degradation. PLLA microspheres released lactic acid at a significantly higher rate than PLGA microspheres.

### Example 4 Cytotoxicity assay

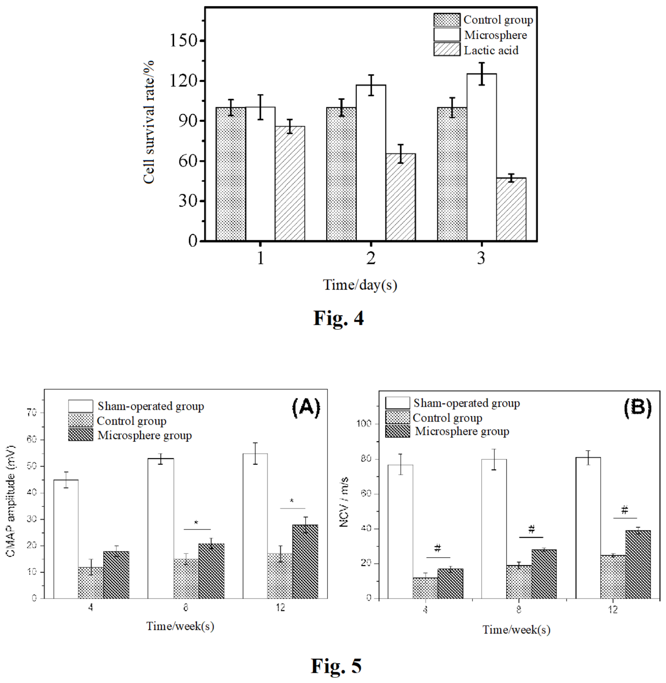

A cell suspension with a concentration of 7×10⁴ was prepared from nerve cells (rat Schwann cells (RSC96)) in the logarithmic growth phase. The cell suspension was seeded in a 96-well plate at a density of 200 µl per well. The culture plate was placed in an incubator and cultured until the cells covered more than 95% of the bottom area of the well. Then, 100 µL of PLLA microspheres prepared in Example 1 at a concentration of 5mg /mL and 5 mg/mL of untreated bulk lactic acid were added to each well of the culture plate. At least 5 parallel wells were set for each sample. The culture plate was then placed in an incubator and incubated in the dark for 24, 48 and 72 hours. Then, 10 µL of LCCK-8 solution was added to each well, and the culture plate was placed in an incubator for 1 h. Then, the optical density (OD) at 450 nm was measured using a microplate reader, and the cell viability was calculated. The results are shown in Fig. 4.

According to the results, the nerve cell survival rate of the microspheres prepared by the method of the present invention was significantly higher than that of the bulk lactic acid. It can be seen that the microspheres prepared by the method of the present invention have higher pharmacological activity compared to the bulk lactic acid. The sustained-release microspheres have a better promoting effect on nerve cells than the bulk lactic acid, which may be because the activity of nerve cells is more sensitive to the concentration of lactic acid.

### Example 5: Promoting nerve injury recovery in vivo

### 1. Establishment of an animal model of spinal cord nerve injury

Healthy SD rats, weighing 220-250g, were randomly divided into 4 groups: a normal saline control group, a low-concentration PLLA microsphere group, a medium-concentration PLLA microsphere group, and a high-concentration PLLA microsphere group, with 6 rats in each group.

Rats were anesthetized by intraperitoneal injection of 3.0% (w/v) sodium pentobarbital (0.2 ml/100 g). After complete anesthesia, the rats were fixed in a prone position. After skin preparation and routine disinfection, a longitudinal incision (2-3 cm) was made along the midline of the back, with the twelfth thoracic vertebra (T12) as the base point. The incision was made from the outside to the inside until the subcutaneous fascia was reached. The dorsal lamina of the rats from T9 to T11 was accurately removed to expose the dura mater of the spinal cord. The middle part of the spinal cord was clamped and hit with an aneurysm clip for 40 s to prepare a model of spinal cord injury (SCI).

### 2. Experimental grouping

Group A: Normal saline, without PLLA microspheres, serving as the control group.

Group B: Low-concentration PLLA microspheres group, normal saline with PLLA microspheres of Example 1, microsphere concentration (10 mg/mL).

Group C: Medium-concentration PLLA microspheres group, normal saline with PLLA microspheres of Example 1, microsphere concentration (50 mg/mL).

Group D: High-concentration PLLA microspheres group, normal saline with PLLA microspheres of Example 1, microsphere concentration (150 mg/mL).

### 3: PLLA microspheres promote nerve injury recovery

Each group was medicated according to body weight at the following times: immediately after injury, 1 hour after injury, 3 hours after injury, 8 hours after injury, 24 hours after injury, 3 days after injury, 7 days after injury, 14 days after injury, 21 days after injury, and 28 days after injury (20 µL of the samples of the control group and the experimental groups were injected epidurally). The recovery of motor function in the lower limbs of the animals was observed. Samples were taken at 8 hours, 24 hours, 7 days, 28 days, and 3 months after injury for gross and pathological examination. The results showed that group C showed significantly better recovery of motor function in the lower limbs than the other groups, while group A, the control group, showed no significant recovery. Both gross and pathological observations showed that groups B and C had the minimal local inflammatory response in spinal cord injury.

### 4. Effects of polylactic acid microspheres on lipid peroxides after acute spinal cord transection injury in rats

15 min after establishing the complete spinal cord transection injury model, the control group was given 83 µL /kg of normal saline at the spinal cord defect site, and the experimental groups include 3 groups, each given 100 µL of the above 3 different concentrations of PLLA microsphere suspension. At 1, 4 and 24 hours after injury, the content of malondialdehyde, a lipid peroxidation product, in the spinal cord tissue proximal and distal to the severed ends was determined by the thiobarbituric acid method. The results are shown in Table 1.

**Table 1. Malondialdehyde content in rat spinal cord tissue (unit: nmol/mg)**

| Group | 1h | 4h | 24h |
|---|---|---|---|
| Normal saline group C | 1.36±0.06 | 7.28±0.15 | 10.23±0.11 |
| Low dose group B 1% | 0.91±0.04 | 6.52±0.13 | 8.52±0.21 |
| Medium dose group A 4% | 0.53±0.03 | 5.46±0.15 | 7.16±0.17 |
| High dose group 12% | 0.59±0.03 | 5.97±0.12 | 7.41±0.09 |

The above experimental results indicate that the PLLA microspheres in the present invention can reduce the level of malondialdehyde in the spinal cord at the severed ends after complete transection injury of the spinal cord, reduce the damage caused by lipid peroxidation, and thus protect the spinal cord tissue. In this experiment, by observing the changes in malondialdehyde content in the spinal cord at the severed ends after complete transection spinal cord injury in rats, it is verified that the drug of this invention provides early protection of the complete transection of the spinal cord, thereby extending the time window for early treatment.

### Example 6: Promoting the regeneration of peripheral nerves in vivo

### 1. Establishment of a sciatic nerve injury model

SD rats were divided into three groups, 10 rats in each group: sham-operated group, control group, and microsphere group. After the rats were anesthetized with isoflurane inhalation, the right sciatic nerve was exposed under aseptic conditions. The sciatic nerve was transected 5 mm proximal to the bifurcation point of the sciatic nerve with a blade and sutured with one stitch using 8-0 absorbable suture. The anastomosis site was then treated according to the grouping, and marked with 10-0 non-absorbable suture. After suturing layer by layer, the rats were fed normally.

### 2. Experimental grouping

Sham-operated group: No nerve transection performed
Control group: After nerve transection, the anastomosis site was sutured with only one stitch using 8-0 absorbable suture.
Microsphere group: After nerve transection, the anastomosis site was sutured with one stitch using 8-0 absorbable suture, and then 10 mg of PLGA microspheres were added, wherein the PLGA microspheres were prepared according to the preparation process in Example 2.

### 3. Assessment of functional recovery after nerve injury

At 4, 8, and 12 weeks after operation, compound muscle action potential (CMAP) and nerve conduction velocity (NCV) were measured using an electromyograph to assess functional recovery following nerve injury. The specific procedure is as follows: the rat was anesthetized by inhaling isoflurane, and the original incision was reopened to expose the sciatic nerve. The receiving electrodes were connected and inserted into the middle of the Achilles tendon and gastrocnemius muscle respectively, and the ground electrode was inserted subcutaneously. The stimulation electrode was adjusted to 10mA, and the distal and proximal ends of the sciatic nerve anastomosis were stimulated respectively. The potentials were recorded, and the CMAP and NCV values were calculated.

CMAP reflects the number of newly generated axons, and the test results are shown in Fig. 5(A) (*p<0.05). It can be seen that at 4 weeks after operation, there was no statistically significant difference between the control group and the microsphere group. Eight weeks after operation, the CMAP in the microsphere group was significantly higher than that in the control group. Twelve weeks after operation, the CMAP in the microsphere group was significantly higher than that in the control group, with its CMAP value reaching 28±3mV. NCV reflects the maturity of myelin sheath. The NCV test results of the samples of the three groups are shown in Fig. 5(B) (#p<0.01). It can be seen that at weeks 4, 8 and 12 after operation, the microsphere group had higher values than the control group, with significant differences. At week 12, the NCV value of the microsphere group reached 39+2m/s. The test results above demonstrate that PLGA microspheres have a significant effect on promoting the regeneration and repair of damaged nerves.

Finally, it should be noted that the above embodiments are only used to illustrate the technical solutions of the present invention and are not intended to limit it. Although the present invention has been described in detail with reference to preferred embodiments, those skilled in the art should understand that modifications or equivalent substitutions can be made to the technical solutions of the present invention without departing from the spirit and scope of the technical solutions of the present invention, and all such modifications or substitutions should be covered within the scope of the claims of the present invention.

## Claims

1. Use of a polylactic acid and a copolymer thereof in the preparation of a drug for promoting nerve growth and repair, wherein the molecular weight of the polylactic acid and the copolymer thereof is 400-300 k Da, and the drug has any one of the following effects 1) to 3):
1) promoting the growth of nerve cells and nerve tissue;
2) promoting repair of nerve cells and nerve tissue;
3) treating or preventing a peripheral nerve injury, a spinal cord injury, peripheral neuropathy, and neuritis;
preferably, the molecular weight is 5000-100 k Da.

2. The use according to claim 1, wherein the polylactic acid and the copolymer thereof are from the following combination: poly-L-lactic acid, poly-D,L-lactic acid, and copolymers comprising L-lactic acid but not D-lactic acid.

3. The use according to claim 2, wherein the crystallinity of the poly-L-lactic acid is 0~85%, preferably 10~40%, and its molecular weight distribution is 1~3, preferably 1.1~1.8.

4. The use according to claim 2, wherein the concentration of L-lactic acid in the poly-D,L-lactic acid is at least 50%; more preferably, the ratio of L-lactic acid to D-lactic acid in the poly-D,L-lactic acid is about 1:1.

5. The use according to claim 2, wherein the concentration of L-lactic acid in the copolymer comprising L-lactic acid but not D-lactic acid is at least 20%;
preferably, the copolymer comprising L-lactic acid but not D-lactic acid includes one or more of poly(lactic acid-glycolic acid) copolymer, poly(lactic acid-ethylene glycol) copolymer, poly(ethylene glycol-lactic acid-glycolic acid) copolymer, polylactic acid-chitosan copolymer, and poly(lactide-caprolactone) copolymer.

6. The use according to claim 1, wherein the polylactic acid and the copolymer thereof exist in the form of a formulation.

7. The use according to claim 6, wherein the formulation includes microspheres, micelles, or gels,
preferably, the microspheres are spherical and have a smooth surface; the particle size distribution of the microspheres is 1-100 µm, preferably 30-60 µm.

8. The use according to claim 6, wherein the formulation further comprises excipients, and the excipients include surfactants and stabilizers;
the surfactant includes one or more of polyethylene glycol, sodium dodecyl sulfonate, Tween, and Span; and the stabilizer includes one or two of carboxymethyl cellulose and mannitol.

9. The use according to claim 6, wherein the formulation further comprises a first active ingredient having a neuroprotective effect, and preferably, the first active ingredient includes one or more of piracetam, aniracetam, oxiracetam, methylcobalamin, adenosylcobalamin, gangliosides, mannitol, edaravone, methylcobalamin, rapamycin, nerve growth factor, and vitamin B.

10. The use according to claim 6, wherein the concentration of L-lactic acid in the polylactic acid and the copolymer thereof in the formulation during at least one dosing period is 5~2500 mmol/L, preferably 100~1200 mmol/L.
